# EUROPEAN PATENT SPECIFICATION

(11) **EP 1 894 598 B1**
(45) Date of publication and mention of the grant of the patent: **10.06.2009**
(21) Application number: 07114233.5
(22) Date of filing: 13.08.2007
(51) Int. Cl.: A61M 39/22, F16K 11/02

(54) **Liquid coinfusion unit**
Einheit zur Koinfusion von Flüssigkeiten
Unité de co-infusion liquide

(30) Priority: 01.09.2006 JP 2006238070
(43) Date of publication of application: 05.03.2008
(73) Proprietor: Covidien AG, 8212 Neuhausen am Rheinfall (CH)
(72) Inventor: Kitani, Ichiro, Shizuoka-ken 437-0004 (JP); Sakai, Yosuke, Shizuoka-ken 437-0004 (JP)
(74) Representative: Körber, Martin Hans

(56) References cited:
- EP-A- 1 627 658
- WO-A-00/40291
- WO-A-03/015851
- US-A- 2 854 027
- US-A- 3 965 910

## Description

### Technical field

The present invention pertains to a liquid coinfusion unit that has a main flow channel for feeding medicinal liquid or another liquid from an upstream tube via a chamber to the downstream tube, and a secondary flow channel for feeding medicinal liquid or another liquid from a mixing tube to the main flow channel.

### Background of the invention

In the prior art, physiological saline, medicinal liquid or the like is fed into the body of the patient by means of a transfusion tube. In such cases, a flow rate adjusting device is used to adjust the flow rate of the medicinal liquid or the like flowing through the transfusion tube (for example, see Japanese Kokai Patent Application No. Sho 62[1987]-172962). Said flow rate adjusting device is composed of an inner wall with its lower portion formed in a valve chamber, a main body composed of two tube joints having through-holes and extending from the two sides of the vacuum chamber to the outside, respectively, and a movable rotatably mounted in the inner wall of the main body. The two tube joints of the main body are formed vertically offset from each other with respect to the central axis, and the lower portion of the movable part is formed as a slope. Consequently, by rotating the movable part, it is possible to open/close the through-hole by changing the size of the opening formed by the through-holes on the two tube joints in the movable part. As a result, it is possible to adjust as desired the flow rate of the medicinal liquid or the like fed from one tube joint via the vacuum chamber to the other tube joint.

US-A-2 854 027 discloses a multi-way stop cock-type valve comprising a first flow channel from an upstream tube to a downstream tube and a second flow channel from a mixing tube to a downstream tube whereby the a closure mechanism is provided which is able to releasably block the first flow channel while keeping the second flow channel open.

### Summary of the invention

However, the aforementioned flow rate adjusting device is associated with certain problems. It is used with transfusion tubes for feeding one type of physiological saline, medicinal liquid or the like into the body of the patient, and it is not used for feeding a plurality of types of physiological saline, medicinal liquid, etc. into the body of the patient. As a result, a liquid coinfusion unit has been developed that can control the supply of a plurality of types of physiological saline, medicinal liquid, etc. to the patient by connecting between or blocking plural transfusion tubes. When said liquid coinfusion unit for feeding a plurality of medicinal liquids, etc. is used to feed one medicinal liquid, etc., if another medicinal liquid, etc., is fed together with the first one, then the other medicinal liquid, etc., will flow back and invade the upstream side of the tube for feeding said first medicinal liquid, etc. In order to prevent said back flow, a liquid coinfusion unit that can switch the flow channel by rotating a valve body has also been developed. However, the process for connecting between or blocking the plurality of tubes is complicated, which is undesirable.

The purpose of the present invention is to solve the aforementioned problems of the prior art by providing a liquid coinfusion unit characterized by the fact that it is possible to prevent the back flow of the medicinal liquid, etc. and to facilitate the operation for connecting or blocking any of the tubes of the plurality of tubes.

In order to realize the aforementioned purpose, the present invention provides a liquid coinfusion unit comprising: a liquid coinfusion unit main body, having an upstream tube and a downstream tube extending outwardly from two respective sides of an interior chamber, and a mixing tube which is formed on an upper part of said chamber and which communicates with a first flow channel that extends from said upstream tube to said downstream tube via said chamber such that a second flow channel extends from said mixing tube to said downstream tube; a closure mechanism arranged to be able to releasably block said first flow channel; and a manipulating element outside of said chamber which is connected to said closure mechanism which can be manipulated to displace said closure mechanism, whereby said closure mechanism is biased towards a state in which said first flow channel is open.

In an aspect of the present invention, the liquid coinfusion unit contains a closure mechanism, which is set in said chamber and can connect/block the portion between said upstream tube and said chamber in said first flow channel by being displaced inside said chamber; and a manipulating element outside of said chamber, which is connected to said open/close part and can be manipulated for the displacement of said open/close part. By manipulating the manipulating element from outside of the chamber, it is possible to block or connect the part between the upstream tube and the chamber in the first flow channel. As a result, by manipulating the manipulating element, it is possible to turn on and off the supply of the medicinal liquid or the like from the upstream tube via the chamber to the downstream tube.

Here, "the displacement of the closure mechanism" means changing the state of the closure mechanism by, e.g., deformation, movement, etc., so that the first flow channel can be opened/closed.

In a further aspect of the present invention, the second flow channel can be connected or blocked. This may be performed, for example, by attaching a rubber stopper in the branched tube to block the second flow channel and piercing the rubber stopper with a cylindrically shaped needle, syringe, etc., to connect the second flow channel. That is, with said liquid coinfusion unit, it is possible to connect or block the portion between said upstream tube and said chamber in said first flow channel from the upstream tube via the chamber to the downstream tube, and it is also possible to block or connect the second flow channel.

As a result, it is possible to feed one type of medicinal liquid or the like to the body of the patient, or to feed two types of medicinal liquids or the like into the body of the patient. Also, by feeding medicinal liquid or the like from the mixing tube to the downstream side of the first flow channel while manipulating the manipulating element to block the first flow channel, said medicinal liquid does not flow back to the upstream side of the first flow channel but flows to the downstream side of the first flow channel. As a result, with the first flow channel blocked, after the required quantity of the medicinal liquid or the like is fed from the mixing tube to the downstream side of the main flow channel, the blocking of the first flow channel can be released, so that the medicinal liquid or the like from the upstream side to the downstream side of the first flow channel can flow together with the medicinal liquid or the like fed from the mixing tube into the body of the patient. As a result, the desired quantity of medicinal liquid or the like fed from the mixing tube can be supplied appropriately and quickly into the body of the patient.

Also, in one aspect of the present invention the liquid coinfusion unit is characterized by the fact that said closure mechanism is energized in the direction of connecting said upstream tube and said chamber in said main flow channel, so that the portion between said upstream tube and said chamber in said first flow channel can only be blocked if said manipulating element has been manipulated.

As a result, for example, when a medicinal liquid or the like is fed from the mixing tube to the first flow channel, by manipulating the manipulating element to block the upstream side of the first flow channel, it is possible to prevent the medicinal liquid or the like fed from the mixing tube from flowing back to the upstream side of the first flow channel, and then, by leaving the manipulating element in this position, it is possible to flow another medicinal liquid or the like from the upstream side to the downstream side of the first flow channel. As a result, when a small quantity of medicinal liquid or the like is to be fed from the mixing tube to the first flow channel, the operation becomes easier, and at the same time, it is possible to feed the medicinal liquid or the like appropriately.

Also, in one aspect of the present invention the liquid coinfusion unit is characterized by the fact that said closure mechanism comprises a deformable part made of a flexible plastic body having portions on either side of said first flow channel and wherein said manipulating element is composed of flexible elastic compressible parts integrated with said deformable part.

As a result, if pressure is applied to deform the compressible parts so that they enter the chamber, the deformable part also deforms, so that the portions of the deformable part where the first flow channel is held come in contact with each other to block the first flow channel. In this case, since the deformable part and compressible parts are flexible elastic bodies, when the pressure applied to the compressible parts is released, the deformable part and the compressible parts recover their original shape so that the first flow channel becomes connected. Also, in this liquid coinfusion unit, it is preferred that a guide part for guiding the displacement (deformation) of said deformable part be arranged within said chamber. As a result, the deformation of the deformable part can take on the prescribed state.

In addition, in one aspect of the present invention the liquid coinfusion unit is characterized by the fact that said closure mechanism comprises a pair of facing pieces arranged on either side of the first flow channel and which extend through side walls of said chamber to the outside, wherein said manipulating part comprises a pair of eleastic manipulating pieces that are connected to said pair of facing pieces and wherein said pair of facing pieces are biased towards a state in which said first flow channel is open.

As a result, by applying pressure to deform the elastic manipulating pieces positioned on the two sides of the chamber so that they enter the chamber and the pair of facing pieces are pressed toward the interior of the chamber, it is possible to bring the ends of the facing pieces on either side of the first flow channel in contact with each other in order to block the first flow channel. In this case, since the constitution is such that the facing pieces are biased towards a state in which the first flow channel is open, as the pressure applied to the elastic manipulating pieces is released, the facing pieces and the elastic manipulating pieces recover their original shape, so that the first flow channel is connected.

Also, in one aspect of the present invention the liquid coinfusion unit is characterized by the fact that said closure mechanism comprises a moving piece, which passes through side walls of said chamber to the exterior and which defines a connecting hole therethrough formed at a prescribed location, said connecting hole being movable from a position in which said connecting hole is aligned with said first flow channel such that said first flow channel is open and a position in which said connecting hole is displaced from said first flow channel such that said first flow channel is closed and wherein an elastic biasing element is provided which biases said moving piece such that said first flow channel is open, and said manipulating element is arranged on an opposite side of said chamber to said biasing element and is arranged to exert a force on said moving piece against the biasing of the elastic biasing element.

As a result, by manipulating the manipulating element positioned outside of the chamber to deform the elastic biasing element, it is possible to move the moving piece so that the connecting hole leaves the position of the first flow channel to block the first flow channel. In this case, since the elastic biasing element energizes the moving piece such that the connecting hole is positioned in the first flow channel, when the manipulating piece is released, the elastic biasing element and the moving piece recover their original shape to connect the first flow channel.

Also, in one aspect of the present invention the liquid coinfusion unit is characterized by the fact that said closure mechanism comprises a moving piece, which passes through side walls of said chamber to the exterior and which defines a connecting slot formed in an upper half thereof at a prescribed location at a side facing toward said upstream tube for connecting said first flow channel via the mixing tube with the downstream tube, and wherein said manipulating element comprises an elastic biasing piece that biases one end of said moving piece so that said connecting slot is aligned with said first flow channel, and a manipulating piece on a second end of said moving piece for moving piece against the biasing of said elastic biasing piece.

As a result, by manipulating the manipulating piece located outside of the chamber to deform the elastic biasing piece, it is possible to move the moving piece so that the connecting slot leaves the position of the first flow channel so that the first flow channel is blocked. In this case, because the elastic biasing piece energizes the moving piece so that the connecting slot is positioned in the first flow channel, when the manipulating piece is released, the elastic biasing piece and the moving piece recover the original shape, the first flow channel is connected.

Also, in one aspect of the present invention the liquid coinfusion unit is characterized by the fact that said closure mechanism comprises an elastic cylindrical body, an interior of which forming a part of said first flow channel, and wherein said manipulating element comprises a pair of compression members that pass through side walls of said chamber from two side portions of said elastic cylindrical body to the outside, wherein, said pair of compression members are movable towards each other so as to close said elastic cylindrical body to fluid flow therethrough.

As a result, by pressing the pair of compression members located on the outside of the chamber on either side, so that they enter the interior of the chamber, it is possible to deform the elastic cylindrical body so that the portions of the two side portions of the elastic cylindrical body are in contact with each other to block the first flow channel. Thus, when the elastic cylindrical body recovers its original shape, the first flow channel is connected.

### Brief description of the figures

Embodiments of the present invention will now be described, by way of example only, with reference to the accompanying drawings in which:
Figure 1 is a plan view illustrating the liquid coinfusion unit in Embodiment 1 of the present invention;
Figure 2 is a side view of the liquid coinfusion unit in a first embodiment;
Figure 3 is a front view of the liquid coinfusion unit in the first embodiment;
Figure 4 is a cross-sectional view along 4-4 in Figure 2;
Figure 5 is a cross-sectional view along 5-5 in Figure 1;
Figure 6 is a cross-sectional view illustrating the state in which the flow channel of the liquid coinfusion unit shown in Figure 4 is blocked;
Figure 7 is a cross-sectional view illustrating the state in which the flow channel of the liquid coinfusion unit shown in Figure 5 is blocked;
Figure 8 is a cross-sectional view illustrating the state in which the flow channel of the liquid coinfusion unit in a second embodiment is connected;
Figure 9 is a cross-sectional view illustrating the state in which the flow channel of the liquid coinfusion unit in the second embodiment is blocked;
Figure 10 is a cross-sectional view illustrating the state in which the flow channel of the liquid coinfusion unit in a third embodiment is connected;
Figure 11 is a cross-sectional view illustrating the state in which the flow channel of the liquid coinfusion unit in the third embodiment is blocked;
Figure 12 is a cross-sectional view illustrating the state in which the flow channel of the liquid coinfusion unit pertaining to a fourth embodiment is connected;
Figure 13 is a cross-sectional view illustrating the state in which the flow channel of the liquid coinfusion unit in the fourth embodiment is blocked:
Figure 14 is a cross-sectional view illustrating the state in which the flow channel of the liquid coinfusion unit in a fifth embodiment is connected;
Figure 15 is a cross-sectional view illustrating the state in which of the flow channel of the liquid coinfusion unit in the fifth embodiment is blocked;
Figure 16 is a cross-sectional view illustrating the state in which the flow channel of the liquid coinfusion unit in a sixth embodiment is connected; and
Figure 17 is a cross-sectional view illustrating the state in which of the flow channel of the liquid coinfusion unit in the sixth embodiment is blocked.

### Preferred embodiments of the invention

### Embodiment 1

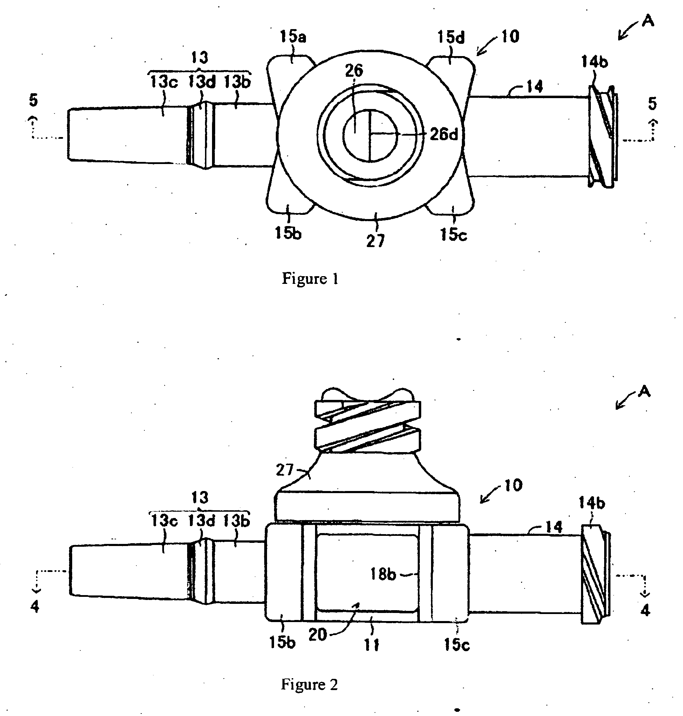

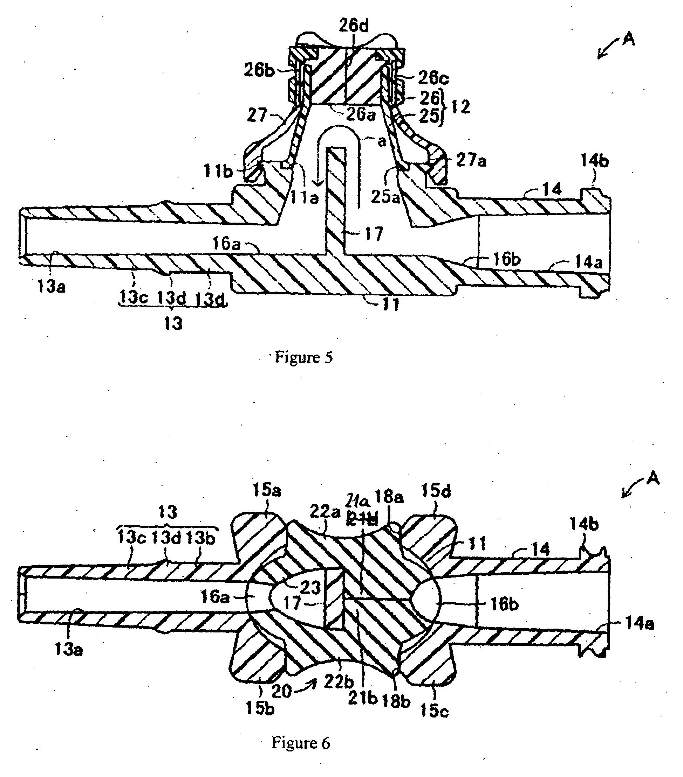

In the following, the liquid coinfusion unit will be explained in more detail as Embodiment 1 of the present invention, referencing. Figures 1-3 illustrate liquid coinfusion unit A of this first embodiment. Liquid coinfusion unit (A) is composed of liquid coinfusion unit main body (10) and open/close manipulating part (20). Said liquid coinfusion unit main body (10) is composed of chamber (11), which is approximately cylindrical in shape and short in length in the axial direction. It is set with its axial direction oriented vertically, mixing tube (12) set above chamber (11) (see Figure 5), and a pair of downstream (13) and upstream (14) tubes formed on the outer peripheral surface of chamber (11) and extending coaxially while maintaining an angle of 180°.

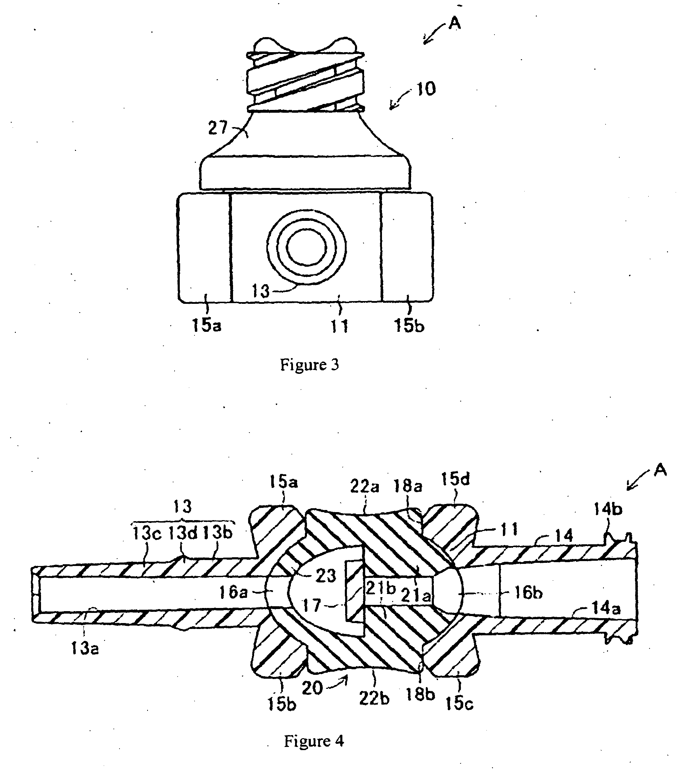

The main body of chamber (11) is formed with a cylindrical shape with the lower end closed and the upper end open and is elliptical in plan view. On its outer peripheral surface, holding protrusions (15a), (15b), (15c), (15d) are formed at intervals along the circumference on the outer peripheral surface. As shown in Figures 4 and 5, two through-holes (16a), (16b) are formed on the portions facing each other near the center in the axial direction of chamber (11). Also, in the portion on chamber (11) corresponding to through-hole (16a), downstream tube (13) is set, and the interior of chamber (11) and flow channel (13a) formed inside downstream tube (13) communicate with each other via through-hole (16a). Also, upstream tube (14) is set on the portion of chamber (11) corresponding to through-hole (16b), and the interior of chamber (11) and flow channel (14a) formed inside upstream tube (14) are connected to each other via through-hole (16b).

Said downstream tube (13) is integrated with chamber (11), and it is composed of base end portion (13b) positioned on the side near chamber (11), and male luer portion (13c) positioned toward the end and formed narrower than base end portion (13b). Also, male luer portion (13c) is gradually tapered from the side near base end portion (13b) toward the end. At the boundary between base end portion (13b) and male luer portion (13c) on the outer peripheral surface of downstream tube (13), protrusion (13d) is formed along the circumference. Also, said upstream tube (14) is integrated with chamber (11), and flow channel (14a) within is tapered shape to have a smaller diameter on the side near through-hole (16b) and with the diameter gradually increasing away from through-hole (16b). Also, threaded portion (14b) for connection is formed on the outer periphery of the opening portion of upstream tube (14).

Also, engagement recess (11 a) for attachment of mixing tube (12) is formed on the inner side of the peripheral edge portion of the upper opening portion of chamber (11), and engaging protrusion (11b) for attachment of cap (27) (explained below) is formed on the outside on the peripheral edge of the upper opening of chamber (11). In said chamber (11), longitudinal wall (17) is set upward from near the center in the front/rear direction (left/right direction in Figures 4 and 5) on the inner bottom surface of chamber (11).

Said longitudinal wall (17) arranged so that it covers the central side portion in the width direction (up/down direction in Figure 4) orthogonal to the direction that connects the portion on the side of flow channel (13a) and the portion on the side of flow channel (14a) inside chamber (11). The upper end extends slightly to the upper side with respect to the upper end opening portion of chamber (11). On the two side portions of chamber (11) (the left/right sides shown in Figure 3), rectangular hole portions (18a), (18b) are formed opposite each other and that pass through from the interior to the outside of chamber (11), and, through said hole portions (18a), (18b), open/close manipulating part (20) is assembled to go from the interior of chamber (11) to the outside.

Said open/close manipulating part (20) is formed as a molding from deformable rubber. It is composed of open/close parts (21a), (21b) as the deformable parts in the present invention set between longitudinal wall (17) and the walls on the side near upstream tube (14) in chamber (11), compressible parts (22a), (22b) connected to the portions corresponding to open/close parts (21a), (21b) and positioned at hole portions (18a), (18b), respectively, and connecting portion (23) that connects the portions on the side of downstream tube (13) between compressible parts (22a), (22b). Here, a prescribed spacing is set between compressible parts (22a), (22b) and the two side portions of longitudinal wall (17) facing them, and said spacing allows deformation of open/close parts (21a), (21b) and compressible parts (22a), (22b) towards the center side in the width direction.

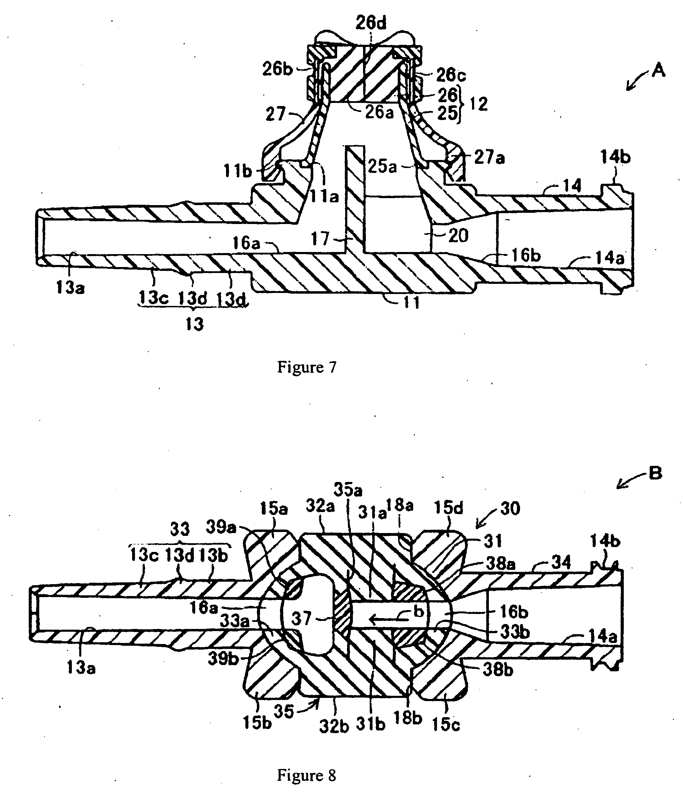

As a result, when compressible parts (22a), (22b) of said open/close manipulating part (20) with said constitution are pressed and deform towards the interior of chamber (11), the opposing surfaces of open/close parts (21a), (21b) come in contact with each other, and as shown in Figures 6 and 7, the flow channel between the interior of chamber (11) and flow channel (14a) of upstream tube (14) is blocked. In this case, longitudinal wall (17) guides the movement of open/close parts (21 a), (21b), and at the same time defines the movement of compressible parts (22a), (22b), so that deformation of compressible parts (22a), (22b) does not become uncontrolled.

On the other hand, when the force applied to press compressible parts (22a), (22b) towards the interior of chamber (11) is released, due to the restoring force of open/close manipulating part (20), open/close parts (21 a), (21 b) separate from each other, and as shown in Figures 4 and 5, the interior of chamber (11) and flow channel (14a) of upstream tube (14) are connected to each other. In this case, as medicinal liquid or the like is fed from the side of upstream tube (14) to the side of chamber (11), after the medicinal liquid or the like flows from flow channel (14a) into the flow channel formed between open/close parts (21 a), (21b) and passes over longitudinal wall (17), it flows from the portion on the side of downstream tube (13) in chamber (11) into flow channel (13a) of downstream tube (13).

In this case, while longitudinal wall (17) prevents the backflow of the medicinal liquid or the like, as the medicinal liquid or the like passes the upper side inside chamber (11), the stagnation of air inside chamber (11) can be prevented. Also, connecting portion (23) is engaged with the inner wall of chamber (11), and open/close manipulating part (20) does not leave the interior of chamber (11) even when open/close manipulating part (20) deforms. Also, the main flow channel of the present invention is formed by flow channel (14a) of upstream tube (14), flow channel (a) that passes from the portion on the side of upstream tube (14) inside chamber (11) over longitudinal wall (17) to the portion on the side of downstream tube (13), and flow channel (13a) of downstream tube (13).

Said mixing tube (12) is composed of approximately cylindrically shaped upstream mixing tube (25) attached on the upper end portion of chamber (11) and rubber stopper (26) made of natural or synthetic rubber and attached on the upper end opening portion of upstream mixing tube (25). Said upstream mixing tube (25) is a short cylindrical body that tapers from a large-diameter lower end to a smaller diameter upper end. It is fixed to chamber (11) by engaging lower end peripheral edge portion (25a) to engagement recess (11a) formed on the interior upper end portion of chamber (11).

Said rubber stopper (26) is composed of thick disk-shaped stopper main body (26a) and approximately ribbon-shaped fixed pieces (26b), (26c) that extend from the two sides of the upper end of stopper main body (26a). Rubber stopper (26) is fixed in position by pressing stopper main body (26a) from the upper end opening portion of upstream mixing tube (25) into upstream mixing tube (25), setting fixed pieces (26b), (26c) are along the outer peripheral surface of upstream mixing tube (25), and fixing cap piece (27) on the outer side of upstream mixing tube (25). That is, a hole is formed at the center of the top surface of cap piece (27), and cap piece (27) is fixed in position as fixed pieces (26b), (26c) press against the sides of upstream mixing tube (25), while the peripheral edge portion on the upper surface of stopper main body (26a) press against the sides of upstream mixing tube (25) to prevent the stopper from being pulled out.

Also, slit (26d) is formed in said rubber stopper (26), so that the inside of upstream mixing tube (25) and the outside of upstream mixing tube (25) are connected to each other to form a secondary flow channel in the present invention. This slit (26d) is closed by the elasticity of rubber stopper (26) when it is not used as the secondary flow channel. On the other hand, when it is used as the secondary flow channel, the secondary flow channel may be formed via a connector or the like by inserting said connector or the like (not shown in the figure). For example, said connector has a male luer portion with an interior flow channel. By inserting the male luer portion into slit (26d) of rubber stopper (26), it is possible to connect the connector and the interior of upstream mixing tube (25) to each other. Also, in this case, the portion between said male luer portion and the peripheral surface of slit (26d) is tightly sealed by the elasticity of rubber stopper (26).

Also, cap piece (27) is attached to the peripheral side of the upper end opening portion of the chamber (11) so that mixing tube (12) is covered, and it is formed as a cylindrical body that tapers from a large diameter on the side of chamber (11) to a gradually smaller diameter at the upper end. Also, said cap piece (27) is formed with an elliptic shape in plan view. Engagement portion (27a) is formed on the lower end of the inner peripheral surface of cap piece (27). This engagement portion (27a) engages with engaging protrusion (11b) formed on the outside of the upper end portion of chamber (11) so that cap piece (27) is fixed on chamber (11).

In this constitution, when a prescribed medicinal liquid is to be fed into the body of a patient (not shown in the figure), the distal end of a transfusion tube (not shown in the figure), the other end of which has a holding needle connected to it that is to be inserted into and fixed on the body of the patient, is connected to downstream tube (13). Also, the male luer portion that is set at the end of the transfusion tube that extends from the container that contains the medicinal liquid to be fed to the patient is connected to upstream tube (14). Then, while the inserted holding needle is fixed to the body of the patient, the medicinal liquid in the container is fed toward and into the body of the patient. When a second medicinal liquid or the like is to be fed to the patient in addition to the medicinal liquid fed from the container or the like, said second medicinal liquid or the like is fed from mixing tube (12) into chamber (11) via the transfusion tube connected to the connector.

That is, as the connector is inserted into slit (26d) of rubber stopper (26) in mixing tube (12), the connector and downstream tube (13) are connected via flow channel (a) inside chamber (11), so that the medicinal liquid or the like is injected from the side of the connector, and the medicinal liquid or the like is fed to the patient. In this case, by pushing compressible parts (22a), (22b) of open/close manipulating part (20) into the interior of chamber (11), as shown in Figures 6 and 7, the portion between open/close parts (21a), (21b) is blocked. As a result, the feeding of the medicinal liquid from the container to flow channel (a) is stopped, and at the same time, the backflow of the medicinal liquid or the like that has been injected from mixing tube (12) into chamber (11) to the upstream side of upstream tube (14) can be prevented, so that the medicinal liquid can reliably flow to the downstream side of downstream tube (13).

Also, after the end of injection of the medicinal liquid or the like from the connector side, the pressure on compressible parts (22a), (22b) is released, and the opposing portions of open/close parts (21a), (21b) are opened so that the medicinal liquid can again be fed from the container to the patient. Also, a small quantity of the medicinal liquid is evacuated from the end of the holding needle before the holding needle is applied to penetrate the body of the patient, where it is and held. As a result, it is possible to evacuate the air in the flow channel to the outside together with the medicinal liquid. Also, as the medicinal liquid, which flows from the side of upstream tube (14) through flow channel (a) inside chamber (11) to the side of downstream tube (13), passes through the interior of chamber (11), it passes over longitudinal wall (17) and the upper side of the interior of chamber (11), so that the stagnation of the air inside chamber (11) can be prevented.

Liquid coinfusion unit A in the present embodiment contains open/close manipulating part (20) composed of open/close parts (21 a), (21b) that open/close flow channel (a) inside chamber (11) and compressible parts (22a), (22b) for blocking open/close parts (21a), (21b). As a result, by applying pressure to compressible parts (22a), (22b), the flow of the medicinal liquid from upstream tube (14) to chamber (11) can be stopped, and then application of pressure to compressible parts (22a), (22b) can be stopped. Consequently, it is possible to feed the medicinal liquid or the like from upstream tube (14) via flow channel (a) of chamber (11) to downstream tube (13).

Also, mixing tube (12), which can form the secondary flow channel, is set on the upper side of chamber (11). Consequently, since the connector is connected to mixing tube (12) and the portion on the side of the connector and chamber (11) are connected to each other so that the medicinal liquid or the like is fed from the secondary flow channel to the main flow channel inside chamber (11), it is possible to feed another medicinal liquid or the like via the secondary flow channel from chamber (11) to downstream tube (13). As a result, it is possible to feed either one type of medicinal liquid or the like or two types of medicinal liquid or the like to the body of the patient.

Also, while said compressible parts (22a), (22b) are in the manipulated state, as the medicinal liquid or the like is fed from the side of mixing tube (12), said medicinal liquid or the like flows to downstream tube (13) on the downstream side of the main flow channel without flowing back to upstream tube (14) on the upstream side. As a result, the appropriate quantity of medicinal liquid or the like fed from the side of mixing tube (12) is not diluted by mixing with the medicinal liquid on the upstream side. Instead, it is fed unchanged to the body of the patient suitably and quickly. Also, only when compressible parts (22a), (22b) are manipulated, is the portion between upstream tube (14) and chamber (11) in the main flow channel blocked. As a result, when a small quantity of the medicinal liquid or the like is fed from the side of mixing tube (12) to the main flow channel, the operation can be performed easily, and at the same time, the medicinal liquid or the like can be fed appropriately. Also, when open/close parts (21a), (21b) are driven (deformed) to open/close, longitudinal wall (17) guides said open/close parts (21a), (21b), so that open/close parts (21a), (21b) deform to the appropriate degree to open/close the flow channel.

### Embodiment 2

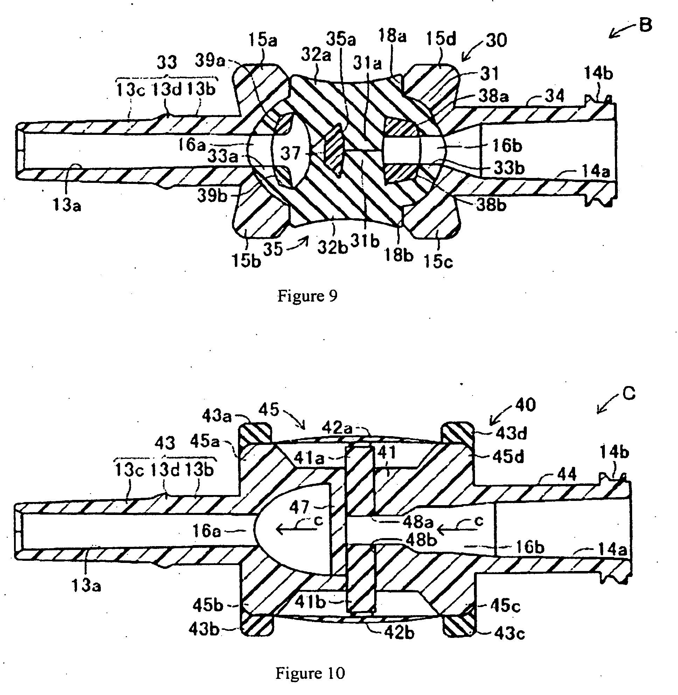

Figures 8 and 9 illustrate liquid coinfusion unit B in Embodiment 2 of the present invention. This liquid coinfusion unit B is composed of liquid coinfusion unit main body (30) and open/close manipulating part (35). Longitudinal wall (37) formed on the bottom surface of chamber (31) contained in liquid coinfusion unit main body (30) has a smaller width than that of longitudinal wall (17) of said liquid coinfusion unit A, and it is formed in an arc-like shape, with the central portion protruding from the two side portions towards the side of upstream tube (34) in a plan view of the surface on the side of longitudinal wall (37) near upstream tube (34).

Also, in the interior portion of chamber (31) on the side of upstream tube (34), a pair of opposing guide pieces (38a), (38b) are formed on either side of flow channel (b), and in the interior portion of chamber (31) on the side of downstream tube (33), a pair of opposing supporting pieces (39a), (39b) are formed on either side of flow channel (b). The shape of the plan view surface of guide pieces (38a), (38b) on the side of downstream tube (33) and the overall shape of the hypothetical extension line (not shown in the figure) connecting them is that of an arc, where the central portion protrudes from the two side portions towards the side of downstream tube (33).

Said open/close manipulating part (35) is composed of open/close parts (31a), (31b) with longitudinal wall (37) arranged inside chamber (31) and opposing guide pieces (38a), (38b) on either side of flow channel (b), compressible parts (32a), (32b) respectively connected to the portions corresponding to said open/close parts (31a), (31b), and connecting parts (33a), (33b) that connect the two side portions of said compressible parts (32a), (32b). The prescribed portions on the inner surfaces of said compressible parts (32a), (32b) are in contact with longitudinal wall (37), guide pieces (38a), (38b) and supporting pieces (39a), (39b), and at the boundary portions (central side portions) of the four locations between open/close parts (31a), (31b) and compressible parts (32a), (32b), slits (35a) are formed extending along the width direction, respectively.

As a result, when pressure is applied to compressible parts (32a), (32b), they respectively deform towards the inside of chamber (31), and open/close parts (31a), (31b) now in contact with each other cause the inner surfaces of slits (35a) to press against longitudinal wall (37) and guide pieces (38a), (38b). As shown in Figure 9, in flow channel (b), the portion between chamber (31) and upstream tube (34) is blocked. In this case, because the surface of longitudinal wall (37) on the side of upstream tube (34) and the surfaces of guide pieces (38a), (38b) on the side of downstream tube (33) are in the form of opposing convex arcs, the facing side portions of open/close parts (31a), (31b) having tapered end portions are in contact with each other. On the other hand, when the force used to push compressible parts (32a), (32b) towards the inside of chamber (31) is released, due to the restoring force of open/close manipulating part (35), open/close parts (31a), (31b) separate, and as shown in Figure 8, connecting the portion in flow channel (b) between chamber (31) and upstream tube (34).

Also, connecting portion (33a) engages with the inner wall of chamber (31) and supporting pieces (39a), (39b), and connecting portion (33b) engages with the inner wall of chamber (31) and guide pieces (38a), (38b). As a result, even when open/close manipulating part (35) is deformed, it cannot escape from the interior of chamber (31). The constitution of the remaining portions of said liquid coinfusion unit B is the same as that of said liquid coinfusion unit A. Thus, the same part numbers are used, and their explanation will not be repeated.

As a result, since longitudinal wall (37) and guide pieces (38a), (38b) for guiding open/close parts (31a), (31b) are set in chamber (31), open/close parts (31a), (31b) remain in an appropriate state of deformation. Also, since the surface of longitudinal wall (37) on the side of upstream tube (34) and the surfaces of guide pieces (38a), (38b) on the side of downstream tube (33) are in the form of opposing convex arcs, and slits (35a) are formed at the boundary portions between open/close parts (31a), (31b) and compressible parts (32a), (32b), the operation for blocking open/close parts (31a), (31b) can be performed smoothly. The remaining features of the operation and effects of this liquid coinfusion unit B are the same as those of said liquid coinfusion unit A.

### Embodiment 3

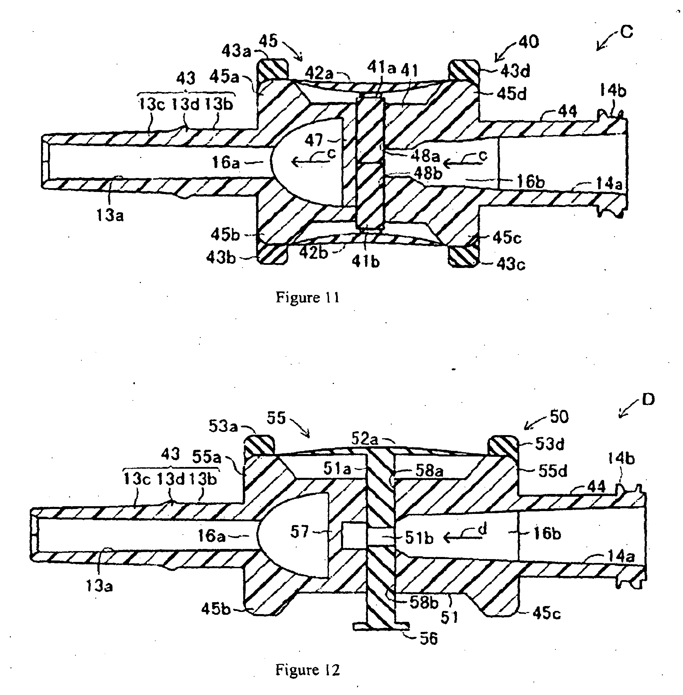

Figures 10 and 11 illustrate liquid coinfusion unit C in Embodiment 3 of the present invention. This liquid coinfusion unit C is composed of liquid coinfusion unit main body (40) and open/close manipulating part (45). Longitudinal wall (47) formed on the bottom surface of chamber (41) contained in liquid coinfusion unit main body (40) is integrated with chamber (41) extending to the two inner walls of chamber (41). Consequently, said longitudinal wall (47) is formed so that the entire area between the front side and the rear side inside chamber (41) in the width direction can be blocked. Also, the part of chamber (41) that forms flow channel c from the portion on the side of upstream tube (44) to the side portion of downstream tube (43) via the upper portion of longitudinal wall (47) forms a small portion with a small end that extends straight with the same diameter as that in approximately the central portion of chamber (41) such that the portion on the side upstream tube (44) is continuous from flow channel (14a) of upstream tube (44).

The portion on the side of downstream tube (43) inside said chamber (41) is formed along the outer shape of chamber (41) and forms an approximately semicircular space in plan view. As a result, a step is formed within chamber (41), in the width direction of the boundary portion between the portion on the side of upstream tube (44) and the portion on the side of downstream tube (43). In this step portion (the portion on the side of upstream tube (44) adjacent to longitudinal wall (47)), continuous hole portions (48a), (48b) are formed from the outer surface of chamber (41) to the inner surface. The width of said hole portions (48a), (48b) is made smaller than the width of hole portions (18a), (18b) of said liquid coinfusion unit A.

Said open/close manipulating part (45) is composed of open/close pieces (41 a), (41b) that are opposing each other and movable through-hole portions (48a), (48b) of chamber (41) with flow channel (c) between them, elastic manipulating pieces (42a), (42b) set connected to the outer side end portions of open/close pieces (41a), (41b), respectively, holding protrusions (45a), (45d) and holding protrusions (45b), (45c) on the two side portions of elastic manipulating pieces (42a), (42b), as well as fixing portions (43a), (43d) and fixing portions (43b), (43c) fixed on said holding protrusions, respectively. Said open/close pieces (41a), (41b) are approximately rod-shaped and can move inside hole portions (48a), (48b) while the surface on the side of longitudinal wall (47) slides on longitudinal wall (47) and is guided by longitudinal wall (47).

Also, said elastic manipulating pieces (42a), (42b) are formed as thin sheets extending in the front/rear direction (left/right direction in Figures 10 and 11), with the inner surface on the side of chamber (41) formed as a plane, and with the outer surface formed as a curved surface with the central portion in the front/rear direction protruding to the outer side with respect to the two side portions. Consequently, the central portions of elastic manipulating pieces (42a), (42b) in the front/rear direction is thicker, and the thickness decreases from the central portion to the two sides. Also, elastic manipulating pieces (42a), (42b) energize the corresponding open/close pieces (41a), (41 b) toward the outside, and the end portions of open/close pieces (41 a), (41b) on the inside of chamber (41) are positioned at the openings of the inner side of hole portions (48a), (48b).

As a result, when elastic manipulating pieces (42a), (42b) are pressed toward the inside of chamber (41) and bent such that the inner surface of elastic manipulating pieces (42a), (42b) protrude towards the side of chamber (41), open/close pieces (41a), (41b) move towards the inside of chamber (41) along the inner surfaces of hole portions (48a), (48b) and longitudinal wall (47). Since the opposing end portions of open/close pieces (41a), (41b) are in contact with each other, as shown in Figure 11, flow channel (c) is blocked. On the other hand, when the force pressing elastic manipulating pieces (42a), (42b) towards the interior of chamber (41) is released, due to the restoring force of elastic manipulating pieces (42a), (42b), said open/close pieces (41a), (41b) separate, and as shown in Figure 10, connect flow channel (c) between chamber (41) and upstream tube (44).

The constitution of the remaining portions of said liquid coinfusion unit C is the same as that of said liquid coinfusion unit A. As a result, the same part numbers as those adopted above are used here and will not be explained again. Thus, because open/close pieces (41 a), (41b) are rod-shaped, the displacement path of open/close pieces (41a), (41b) will be correct. Also, because the manipulatable surfaces of elastic manipulating pieces (42a), (42b) are selectively large, operation becomes easier. The remaining features of the operation and effects of this liquid coinfusion unit C are the same as those of said liquid coinfusion unit A.

### Embodiment 4

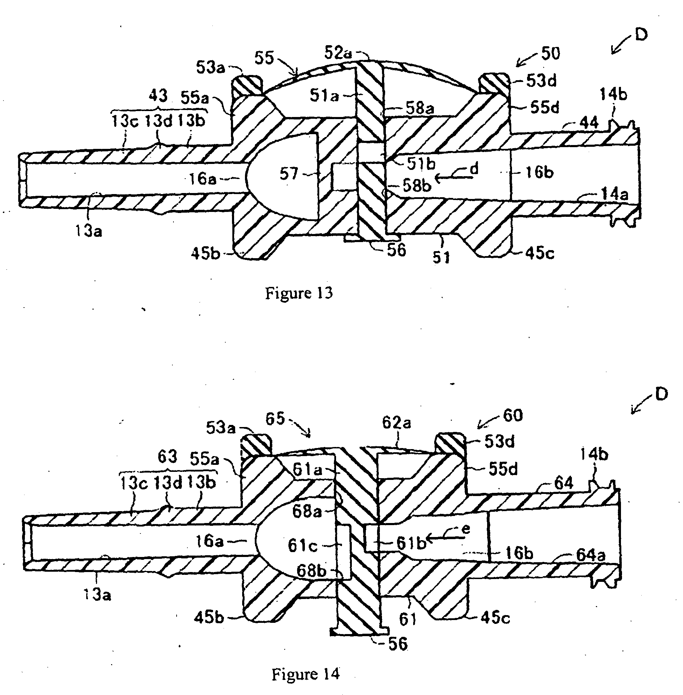

Figures 12 and 13 illustrate liquid coinfusion unit D in Embodiment 4 of the present invention. Liquid coinfusion unit D is composed of liquid coinfusion unit main body (50) and open/close manipulating part (55). Longitudinal wall (57) of chamber (51) contained in liquid coinfusion unit main body (50) is set in the front side portion with respect to the central area in the front/rear direction of chamber (51). Said longitudinal wall (57) extends to the two inner walls of chamber (51), and it is integrated with chamber (51). Also, on the portion with a certain spacing from longitudinal wall (57) in the central portion in the front/rear direction of chamber (51), continuous hole portions (58a), (58b) are formed from the inner surface to the outer surface of chamber (51).

Said open/close manipulating part (55) is composed of moving piece (51 a) movably arranged through hole portions (58a), (58b) of chamber (51), elastic resetting piece (52a) connected to one end of moving piece (51a), fixing portions (53a), (53d) that fix the two side portions of elastic resetting piece (52a) on convex holding parts (55a, (55b), and manipulating piece (56) connecting to and the other end of moving piece (51a). Connecting hole (51b) that forms a portion of flow channel (d) is formed in approximately the central portion of the longitudinal direction (the width direction of chamber (51)) of moving piece (51a). Also, said elastic resetting piece (52a) is formed as the same part as said elastic manipulating piece (42a), and moving piece (51 a) is supported so that connecting hole (51b) is located inside flow channel (d).

As a result, as manipulating piece (56) is pushed towards the inside of chamber (51) and moving piece (51a) is driven to move so that it protrudes to the outside of elastic resetting piece (52a), connecting hole (51b) moves inside hole portion (58a), and as shown in Figure 13, flow channel (d) is blocked by the portion of moving piece (51a) outside of connecting hole (51b). In this case, because elastic resetting piece (52a) is bent towards the outside, if the force for pressing manipulating piece (56) towards the inside of chamber (51) is released, due to the restoring force of elastic resetting piece (52a), moving piece (51a) moves to its original position. As a result, as shown in Figure 12, connecting hole (51b) is positioned inside flow channel (d), and flow channel (d) in chamber (51) is connected.

Also, no fixing portions corresponding to fixing portions (43b), (43c) of liquid coinfusion unit C are set for this liquid coinfusion unit D. The constitution of the remaining portions of liquid coinfusion unit D is the same as that of said liquid coinfusion unit C. As a result, the same part numbers as those adopted above are used, and will not be explained again. As a result, the operation for blocking flow channel (d) becomes simpler since only manipulating piece (56) must be pressed to one side. The remaining features of said liquid coinfusion unit D are the same as those of said liquid coinfusion unit C.

### Embodiment 5

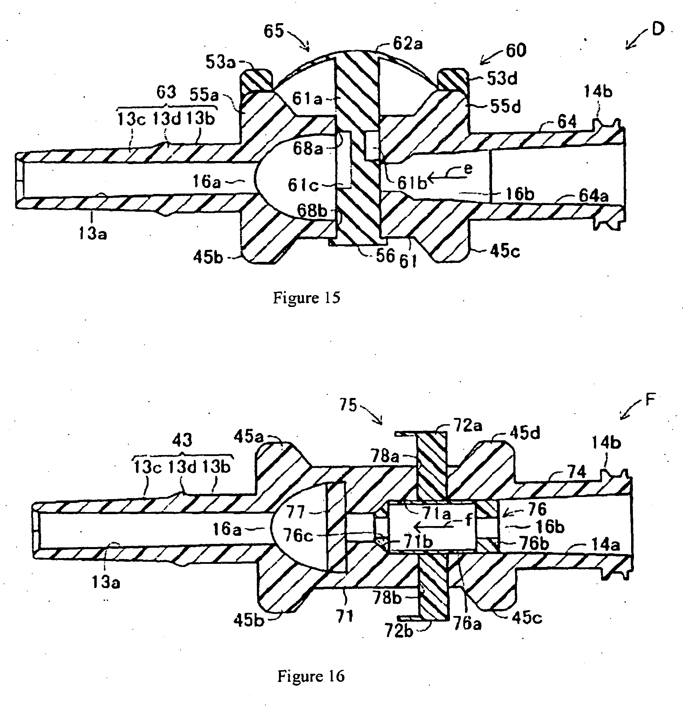

Figures 14 and 15 illustrate liquid coinfusion unit E in Embodiment 5 of the present invention. For said liquid coinfusion unit E, no longitudinal wall is set on chamber (61) of liquid coinfusion unit main body (60). Instead, in the central portion in the front/rear direction of chamber (61), hole portions (68a), (68b) are formed with a larger diameter and length than those of hole portions (58a), (58b) of said liquid coinfusion unit D. Also, the width of the portion on the side of downstream tube (63) in the interior of chamber (61) is extended up to the portion where it crosses hole portions (68a), (68b), and the width of the portion on the side of upstream tube (64) in the interior of chamber (61) is reduced up to the portion where it crosses hole portions (68a), (68b).

Also, the width of moving piece (61a) of open/close manipulating part (65) is set such that it fits the width of hole portions (68a), (68b). At approximately the central portion in the longitudinal direction (width direction of chamber (61)) of moving piece (61a), connecting slots (61b), (61c) are formed as a portion of flow channel (e). Said connecting slot (61b) is set at approximately the center in the width direction on the surface on the side of moving piece (61a) near upstream tube (64), and it connects flow channel (64a) of upstream tube (64) and the upper side portion in chamber (61) in flow channel (e).

Also, connecting slot (61c) is set approximately at the center in the width direction on the surface of moving piece (61a) on the side of downstream tube (63), and the width of connecting slot (61c) is set to about twice the width of connecting slot (61b). Also, elastic resetting piece (62a) supports moving piece (61a) so that connecting slot (61b) is positioned inside flow channel (e). The features of the constitution of the remaining portions of said liquid coinfusion unit E are the same as those of said liquid coinfusion unit D. As a result, the same part numbers as those adopted above are used here and will not be explained again.

Said elastic cylindrical body (76) is made of molded rubber and is composed of thin-walled elastic cylindrical portion (76a), thick-walled upstream annular portion (76b) connected to the upstream end of elastic cylindrical portion (76a) and with a hole formed at the center, and thick-wall downstream ring portion (76c) connected to the downstream end of elastic cylindrical portion (76a) and with a hole formed at the center. On the outer peripheral surface of downstream annular portion (76c), a tapered surface is formed along tapered portion (71b) of hole portion (71a) for forming the flow channel. Also, on the portion of chamber (71) corresponding to approximately the central portion in the axial direction of elastic cylindrical body (76) (the portion of chamber (71) on the side of upstream tube (74) with respect to the central portion in the front/rear direction), continuous hole portions (78a), (78b) are formed from the inner surface of chamber (71) to the outer surface. Also, from the two side portions of elastic cylindrical body (76), a pair of compression parts (72a), (72b) pass through-hole portions (78a), (78b) and extend to the outside.

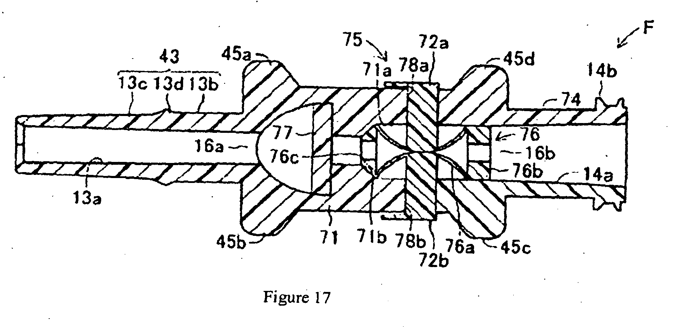

Each of compression parts (72a), (72b) is formed from an approximately L-shaped piece with the end portion on the outside wider so as to facilitate the pressing operation. As a result, when compression parts (72a), (72b) are pressed towards the inside of chamber (71) and the opposing side surfaces of elastic cylindrical body (76) are pressed, as shown in Figure 17, the central portion of elastic cylindrical portion (76a) deforms that flow channel (f) is blocked. On the other hand, when the force for pushing compression parts (72a), (72b) towards the inner side of chamber (71) is released, due to the restoring force of elastic cylindrical portion (76a), elastic cylindrical body (76) recovers its original shape, and as shown in Figure 16, flow channel (f) in elastic cylindrical body (76) is connected. The features of the remaining portions of liquid coinfusion unit F are the same as those of said liquid coinfusion unit C. Consequently, the same part numbers as those adopted above are used here and will not be explained again. As a result, the same operation and effects as those of liquid coinfusion units A, etc. in said embodiments can be realized.

The liquid coinfusion unit of the present invention is not limited to the embodiments described, and appropriate modifications can be performed. For example, in said embodiments, slit (26d) is formed on rubber stopper (26), and a male luer portion is inserted into slit (26d) to connect the connector to mixing tube (12). However, one a scheme can also be used in which instead of the connector, the male luer portion of cylinder or a needle of syringe is inserted into the rubber stopper. When a syringe needle is inserted, there is no need to set slit (26d) on rubber stopper (26). Also, in said Embodiment 5, for liquid coinfusion unit E, two connecting slots, that is, connecting slots (61b), (61c), are set on moving piece (61a). However, one may also adopt a scheme in which only connecting slot (61b) is formed. In addition, appropriate changes may be adopted for the shape and material of the other portions that form the liquid coinfusion unit.

## Claims

1. A liquid coinfusion unit (A) comprising:
a liquid coinfusion unit main body (10), having an upstream tube (14) and a downstream tube (13) extending outwardly from two respective sides of an interior chamber (11), and a mixing tube (12) which is formed on an upper part of said chamber (11) and which communicates with a first flow channel that extends from said upstream tube (14) to said downstream tube (13) via said chamber (11) such that a second flow channel extends from said mixing tube (12) to said downstream tube (13);
a closure mechanism arranged to be able to releasably block said first flow channel; and
a manipulating element outside of said chamber which is connected to said closure mechanism which can be manipulated to displace said closure mechanism,
**characterized in that**
said closure mechanism is biased towards a state in which said first flow channel is open.

2. The liquid coinfusion unit (A) according to Claim 1 wherein said closure mechanism comprises a deformable part made of a flexible elastic body having potions (21a, 21b) on either side of said first flow channel and wherein said manipulating element is composed of flexible elastic compressible parts (22a, 22b) integrated with said deformable part.

3. The liquid coinfusion unit (A) according to Claim 2 wherein said liquid coinfusion unit further comprises a guide part (17) for guiding said deformable part inside said chamber (11).

4. The liquid coinfusion unit (C) according to Claim 1 wherein said closure mechanism comprises a pair of facing pieces (41 a, 41b) arranged on either side of the first flow channel and which extend through side walls of said chamber (41) to the outside, wherein said manipulating part comprises a pair of elastic manipulating pieces (42a, 42b) that are connected to said pair of facing pieces (41 a, 41 b) and wherein said pair of facing pieces (41 a, 41 b) are biased towards towards a state in which said first flow channel is open.

5. The liquid coinfusion unit (D) according to Claim 1 wherein said closure mechanism comprises a moving piece (51a), which passes through side walls of said chamber (51) to the exterior and which defines a connecting hole (5 1 b) therethrough formed at a prescribed location, said connecting hole (51b) being movable from a position in which said connecting hole (51b) is aligned with said first flow channel such that said first flow channel is open and a position in which said connecting hole (51b) is displaced from said first flow channel such that said first flow channel is closed and wherein an elastic biasing element (52a) is provided which biases said moving piece (51a) such that said first flow channel is open, and said manipulating element (56) is arranged on an opposite side of said chamber (51) to said biasing element (52a) and is arranged to exert a force on said moving piece (51a) against the biasing of the elastic biasing element (52a).

6. The liquid coinfusion unit (E) according to Claim 1 wherein said closure mechanism comprises a moving piece (61a), which passes through side walls of said chamber (61) to the exterior and which defines a connecting slot (61b) formed in an upper half thereof at a prescribed location at a side facing toward said upstream tube (64) for connecting said first flow channel via the mixing tube with the downstream tube (63), and wherein said manipulating element comprises an elastic biasing piece (62a) that biases one end of said moving piece (61a) so that said connecting slot (61b) is aligned with said first flow channel, and a manipulating piece (56) on a second end of said moving piece (61a) for moving piece against the biasing of said elastic biasing piece (62a).

7. The liquid coinfusion unit (F) according to Claim 1 wherein said closure mechanism comprises an elastic cylindrical body (76), an interior of which forming a part of said first flow channel, and wherein said manipulating element comprises a pair of compression members (72a, 72b) that pass through side walls of said chamber (71) from two side portions of said elastic cylindrical body (76) to the outside, wherein, said pair of compression members (72a, 72b) are movable towards each other so as to close said elastic cylindrical body (76) to fluid flow therethrough.

## Patentansprüche

1. Koinfusionseinheit für Flüssigkeiten (A), die aufweist
einen Hauptkörper (10) einer Koinfusionseinheit für Flüssigkeiten mit einem vorgeordneten Rohr und einem nachgeordneten Rohr (13), die von zwei zugehörigen Seiten einer Innenkammer (11) nach außen verlaufen, und einem Mischrohr (12), das auf einem oberen Teil der Kammer (11) ausgebildet ist und das in Verbindung mit einem ersten Strömungskanal steht, der von dem vorgeordneten Rohr (14) zum nachgeordneten Rohr (13) über die Kammer (11) verläuft, so dass ein zweiter Strömungskanal von dem Mischrohr (12) zum nachgeordneten Rohr (13) verläuft;
einen Schließmechanismus, der ausgebildet ist, um den ersten Strömungskanal lösbar versperren zu können; und
ein Verstellelement außerhalb der Kammer, das mit dem Schließmechanismus verbunden ist, das verstellt werden kann, um den Schließmechanismus zu versetzen,
**dadurch gekennzeichnet, dass**
der Schließmechanismus in Richtung eines Zustands vorgespannt ist, in dem der erste Strömungskanal offen ist.

2. Koinfusionseinheit für Flüssigkeiten (A) gemäß Anspruch 1, wobei der Schließmechanismus ein verformbares Teil aus einem flexiblen elastischen Körper mit Abschnitten (21a, 2 1 b) auf beiden Seiten des ersten Strömungskanals umfasst und wobei das Verstellelement aus flexiblen, elastisch komprimierbaren Teilen (22a, 22b) besteht, die mit dem verformbaren Teil einstückig sind.

3. Koinfusionseinheit für Flüssigkeiten (A) gemäß Anspruch 2, wobei die Koinfusionseinheit für Flüssigkeiten weiterhin ein Führungsteil (17) zur Führung des verformbaren Teils in der Kammer (11) aufweist.

4. Koinfusionseinheit für Flüssigkeiten (C) gemäß Anspruch 1, wobei der Schließmechanismus ein Paar von einander zugewandten Teilstücken (41a, 41b) aufweist, die an beiden Seiten des ersten Fließkanals angeordnet sind und die durch die Seitenwände der Kammer (41) nach außen verlaufen, wobei das Verstellteil ein Paar elastischer Verstellteilstücke (42a, 42b) aufweist, die mit dem Paar einander zugewandter Teilstücke (41a, 41b) verbunden sind und wobei das Paar der einander zugewandten Teilstücke (41 a, 41b) in Richtung eines Zustands vorgespannt ist, in dem der erste Strömungskanal offen ist.

5. Koinfusionseinheit für Flüssigkeiten (D) gemäß Anspruch 1, wobei der Schließmechanismus ein bewegbares Teilstück (51a) umfasst, das durch die Seitenwände der Kammer (51) nach außen verläuft und das ein hindurchgehendes Verbindungsloch (5 1 b) definiert, dass an einer bestimmten Position ausgebildet ist, wobei das Verbindungsloch (51b) bewegbar ist von einer Position, in der das Verbindungsloch (51b) mit dem ersten Strömungskanal ausgerichtet ist, so dass der erste Strömungskanal offen ist, und einer Position, in der das Verbindungsloch (51b) von dem ersten Strömungskanal versetzt ist, so dass der erste Strömungskanal geschlossen ist, und wobei ein elastisches Vorspannelement (52a) vorgesehen ist, das das bewegbare Teilstück (51 a) vorspannt, so dass der erste Strömungskanal offen ist, und das Verstellelement (56) auf einer abgewandten Seite der Kammer (51) zum Vorspannelement (52a) angeordnet ist, und angeordnet ist, um eine Kraft auf das bewegbare Teilstück (51a) gegen die Vorspannung des elastischen Vorspannelements (52a) auszuüben.

6. Koinfusionseinheit für Flüssigkeiten (E) gemäß Anspruch 1, wobei der Schließmechanismus ein bewegbares Teilstück (61 a) umfasst, das durch die Seitenwände der Kammer (61) nach außen verläuft und einen Verbindungsschlitz (61b) definiert, der in einer oberen Hälfte davon an einer bestimmten Position an einer dem vorgeordneten Rohr (64) zugewandten Seite ausgebildet ist, um den ersten Strömungskanal über das Mischrohr mit dem nachgeordneten Rohr (63) zu verbinden, und wobei das Verstellelement ein elastisches Vorspannteilstück (62a), das ein Ende des bewegbaren Teilstücks (61 a) vorspannt, so dass der Verbindungsschlitz (61 b) mit dem ersten Strömungskanal ausgerichtet ist, und ein Verstellteil (56) auf einem zweiten Ende des bewegbaren Teilstücks (61a) aufweist, um das Teilstück gegen die Vorspannung des elastischen Vorspannteilstücks (62a) zu bewegen.

7. Koinfusionseinheit für Flüssigkeiten (F) gemäß Anspruch 1, wobei der Schließmechanismus einen elastischen zylinderförmigen Körper (76) aufweist, dessen Innenseite einen Teil des ersten Strömungskanals bildet, und wobei das Verstellelement ein Paar von Komprimierungselementen (72a, 72b) aufweist, die durch die Seitenwände der Kammer (71) von zwei Seitenabschnitten des elastischen zylindrischen Körpers (76) nach außen verlaufen, wobei das Paar aus Komprimierungselementen (72a, 72b) zueinander bewegbar ist, um so den elastischen zylinderförmigen Körper (76) für den Durchfluss von Flüssigkeit zu verschließen.

## Revendications

1. Unité de co-infusion de liquides (A) comprenant:
un corps principal (10) de l'unité de co-infusion de liquides, ayant un tube amont (14) et un tube aval (13) s'étendant vers l'extérieur depuis deux côtés respectifs d'une chambre intérieure (11), et un tube de mélange (12) qui est formé sur une partie supérieure de ladite chambre (11) et qui communique avec un premier canal d'écoulement qui s'étend dudit tube amont (14) audit tube aval (13) par ladite chambre (11) de telle sorte qu'un deuxième canal d'écoulement s'étend dudit tube de mélange (12) audit tube aval (13);
un mécanisme de fermeture agencé pour pouvoir bloquer relâchablement ledit premier canal d'écoulement; et
un élément de manipulation à l'extérieur de ladite chambre qui est relié audit mécanisme de fermeture qui peut être manipulé pour déplacer ledit mécanisme de fermeture,
**caractérisée en ce que** ledit mécanisme de fermeture est sollicité vers un état dans lequel ledit premier canal d'écoulement est ouvert.

2. Unité de co-infusion de liquides (A) selon la revendication 1, dans laquelle ledit mécanisme de fermeture comprend une partie déformable réalisée en un corps élastique flexible ayant des portions (21a, 21b) sur chaque côté dudit premier canal d'écoulement, et dans laquelle ledit élément de manipulation est composé de parties compressibles élastiques flexibles (22a, 22b) intégrées avec ladite partie déformable.

3. Unité de co-infusion de liquides (A) selon la revendication 2, dans laquelle ladite unité de co-infusion de liquides comprend en outre une partie de guidage (17) pour guider ladite partie déformable à l'intérieur de ladite chambre (11).

4. Unité de co-infusion de liquides (C) selon la revendication 1, dans laquelle ledit mécanisme de fermeture comprend une paire de pièces se faisant face (41a, 41b) agencées de chaque côté du premier canal d'écoulement et qui s'étendent à travers les parois latérales de ladite chambre (41) vers l'extérieur, dans laquelle ladite partie de manipulation comprend une paire de pièces de manipulation élastiques (42a, 42b) qui sont reliées à ladite paire de pièces se faisant face (41a, 41b), et dans laquelle ladite paire de pièces se faisant face (41a, 41b) est sollicitée dans un état dans lequel ledit premier canal d'écoulement est ouvert.

5. Unité de co-infusion de liquides (D) selon la revendication 1, dans laquelle ledit mécanisme de fermeture comprend une pièce mobile (51a), qui passe à travers des parois latérales de ladite chambre (51) vers l'extérieur et qui définit un trou de connection (51b) à travers celle-ci formé à un emplacement prescrit, ledit trou de connection (51b) étant déplaçable d'une position dans laquelle ledit trou de connection (51b) est aligné avec ledit premier canal d'écoulement de telle sorte que ledit premier canal d'écoulement est ouvert, et une position dans laquelle ledit trou de connection (51b) est déplacé dudit premier canal d'écoulement de telle sorte que ledit premier canal d'écoulement est fermé, et dans laquelle un élément de sollicitation élastique (52a) est réalisé qui sollicite ladite pièce mobile (51a) de telle sorte que ledit premier canal d'écoulement est ouvert, et ledit élément de manipulation (56) est agencé sur un côté opposé de ladite chambre (51) audit élément de sollicitation (52a) et est agencé pour exercer une force sur ladite pièce mobile (51a) contre la sollicitation de l'élément de sollicitation élastique (52a).

6. Unité de co-infusion de liquides (E) selon la revendication 1, dans laquelle ledit mécanisme de fermeture comprend une pièce mobile (61a), qui passe à travers des parois latérales de ladite chambre (61) vers l'extérieur et qui définit une fente de connection (61b) ménagée dans une moitié supérieure de celle-ci à un emplacement prescrit à un côté orienté vers ledit tube amont (64) pour la connection dudit premier canal d'écoulement par le tube de mélange au tube aval (63), et dans laquelle ledit élément de manipulation comprend une pièce de sollicitation élastique (62a) qui sollicite une extrémité de ladite pièce mobile (61a) de telle sorte que ladite fente de connection (61b) est alignée avec ledit premier canal d'écoulement, et une pièce de manipulation (56) sur une seconde extrémité de ladite pièce mobile (61a) pour déplacer la pièce contre la sollicitation de ladite pièce de sollicitation élastique (62a).

7. Unité de co-infusion de liquides (F) selon la revendication 1, dans laquelle ledit mécanisme de fermeture comprend un corps élastique cylindrique (76), dont un intérieur forme une partie dudit premier canal d'écoulement, et dans laquelle ledit élément de manipulation comprend une paire d'éléments de compression (72a,72b) qui passent à travers des parois latérales de ladite chambre (71) depuis deux portions latérales dudit corps élastique cylindrique (76) vers l'extérieur, où les deux éléments de compression (72a, 72b) sont déplaçables l'un vers l'autre de manière à fermer ledit corps élastique cylindrique (76) à un écoulement de fluide à travers celui-ci.
